# EUROPEAN PATENT SPECIFICATION

(11) **EP 3 261 699 B1**
(45) Date of publication and mention of the grant of the patent: **03.04.2019**
(21) Application number: 16710782.0
(22) Date of filing: 26.02.2016
(51) Int. Cl.: A61M 16/00, A61B 6/00, A61N 5/10, A61B 17/00, G16H 20/40

(54) **VENTILATOR SYSTEM**
VENTILATORSYSTEM
SYSTÈME DE VENTILATEUR

(30) Priority: 27.02.2015 GB 201503306
(43) Date of publication of application: 03.01.2018
(73) Proprietor: University Hospitals Birmingham NHS, Edgbaston, West Midlands B15 2GW (GB); The University of Birmingham, Edgbaston, Birmingham B15 2TT (GB)
(72) Inventor: PARKES, Michael, Birmingham West Midlands B15 2TT (GB); GREEN, Stuart, Birmingham West Midlands B15 2GW (GB); CLUTTON-BROCK, Thomas, Birmingham West Midlands B15 2GW (GB)
(74) Representative: Appleyard Lees IP LLP
(86) International application number: PCT/GB2016/050511
(87) International publication number: WO 2016/135509

(56) References cited:
- US-A1- 2001 014 772
- US-A1- 2005 056 283
- US-A1- 2014 066 749

## Description

### Field of the Invention

The present invention relates to ventilator systems, and to associated methods using ventilator systems.

### Background to the Invention

Techniques such as medical imaging and radiotherapy require movement of a patient to be minimized, so as to maximize success of the technique. For example, voluntary or involuntary movement of a patient during CT or MRI imaging will degrade a quality of a resulting image. Likewise, breathing movements of a conscious, unsedated patient during radiotherapy, for example with a LINAC, may reduce an effectiveness of the radiotherapy and/or lead to damage to healthy tissue.

These problems are encountered particularly when the techniques are being performed on the torso, for example when associated with treatment for breast or lung cancer. Chest wall and diaphragm movements associated with breathing are not always predictable. Methods of addressing these problems include using markers to track movement, and voluntary breath holding. Markers may move from their initial positions, and tracking markers is not necessarily determinative of the exact position of the associated tissue of interest. Current techniques for breath holding may only provide a short, e.g. 20 to 30 second time window without significant rhythmic breathing movement. As such these techniques are not usually suitable for, for example, the very young, the elderly or those with reduced lung function or for prolonged "beam on" times during radiotherapy, for example prolonged for a number of minutes.

US 2005/056283 A1 describes a method for integrating a ventilator device with a monitoring device and other medical devices that allows the function of the monitoring device and medical devices to automatically change based on initiation of a respiratory therapy procedure. US 2001/014772 A1 describes a method and apparatus for improving the efficacy of a medical treatment or diagnostic procedure by coordinating such treatment or procedure with a patient's breathing cycle. US 2014/0066749 A1 describes a medical apparatus includes: a breathing device comprising an air pressure generator for generating air pressure, a tube for delivering the air pressure to a patient, and a sensor configured to sense a characteristic associated with a breathing of the patient; and a processing unit configured to receive an output from the sensor, and generate a control signal for controlling a medical device based at least in part on the output from the sensor.

Example embodiments of the present invention aim to address at least one of the issues identified above, or related issues.

### Summary of the Invention

According to the present invention there is provided an apparatus and method as set forth in the appended claims. Other features of the invention will be apparent from the dependent claims, and the description which follows.

According to an aspect of the invention, there is provided a ventilator system comprising:
a ventilator operable to provide forced mechanical ventilation;
a breathing pattern controller configured to operate the ventilator according to a breathing pattern stored therein; and
a breathing pattern communication unit operable to in use transmit a trigger signal, according to the breathing pattern;
wherein the breathing pattern controller is configured to cause the ventilator to actively regulate chest deflation according to the breathing pattern;
wherein the breathing pattern communication unit is operable to in use transmit the trigger signal, according to the breathing pattern, such that the trigger signal is synchronised with the breathing pattern at a point where the breathing pattern controller operates the ventilator to impose a counter inflation to prevent chest deflation in a recipient of the ventilation.

In an example embodiment, the breathing pattern controller is configured to operate the ventilator according to a breathing pattern selected from a plurality of breathing patterns. In an example embodiment, the breathing pattern controller is configured to operate the ventilator according to breathing feedback received from a recipient of the ventilation by adjusting the breathing pattern in response to the breathing feedback.

In an example embodiment, the ventilator system comprises an associated device, and the breathing pattern communication unit is configured to transmit a trigger signal to the associated device. In an example embodiment, the breathing pattern communication unit is configured to transmit a trigger signal to an associated device that comprises a medical device. In an example embodiment, the ventilator system comprises an associated device, and the breathing pattern communication unit is configured to transmit the breathing pattern to the associated device.

In an example embodiment, the breathing pattern communication unit is configured to transmit the breathing pattern to an associated device that comprises a medical device. In an example embodiment, the associated device comprises an imaging device. In an example embodiment, the associated device comprises a radiotherapy device. In an example embodiment, the associated device is a surgical device. In an example embodiment, the associated device comprises for example, a CT scanner, a MRI device, a LINAC radiotherapy device, or a robotic radiosurgery device.

In an example embodiment, the associated device is arranged in use to receive the trigger signal and/or breathing pattern and to operate in response thereto. In an example embodiment, the breathing pattern communication unit is arranged to receive feedback from an associated device, for example patient treatment/imaging feedback, or breathing monitoring feedback, and to communicate the same to the breathing pattern controller, or to a ventilator in the ventilator system.

In an example embodiment the breathing pattern communication unit is operable to in use transmit a trigger signal, according to the breathing pattern, such that the trigger signal is synchronised with the breathing pattern and transmitted at a predetermined point in the breathing pattern. In an example embodiment the breathing pattern communication unit is operable to in use transmit a trigger signal, according to the breathing pattern, such that the trigger signal is synchronised with the breathing pattern and transmitted at a predetermined point in the breathing pattern on a cyclical basis. In an example embodiment the breathing pattern communication unit is operable to in use transmit a trigger signal, according to the breathing pattern, such that the trigger signal is synchronised with the breathing pattern and transmitted at a predetermined point in the breathing pattern that is set with reference to the start of inhalation in the breathing pattern,

In an example embodiment the breathing pattern communication unit is operable to in use transmit a trigger signal, according to the breathing pattern, such that the trigger signal is synchronised with the breathing pattern at a point where the breathing pattern controller operates the ventilator to hold the recipient of ventilation still. In an example embodiment the breathing pattern communication unit is operable to in use transmit a trigger signal, according to the breathing pattern, such that the trigger signal is synchronised with the breathing pattern at a point where the breathing pattern controller operates the ventilator to impose a counter inflation to prevent chest deflation in the recipient of the ventilation during a period of breath-holding, for example during prolonged breath-holding according to the breathing pattern.

In an example embodiment, the associated device is arranged in use to receive the trigger signal and/or breathing pattern and to operate in response thereto, in accordance with movement of the recipient of the ventilation that is produced by operation of the ventilator. In this way, an imaging or radiotherapy treatment or surgical procedure performed using the associated device in the system may be coordinated or synchronized with breathing of a recipient of ventilation. By controlling the breathing of the recipient of the ventilation using the ventilation system the performance of the associated device may be improved, since operation of the associated device may take place at optimum points in the breathing pattern. In an example embodiment, in the ventilator system, regularity and/or a depth of breathing under ventilation becomes relatively predictable so that the trigger signal may be coordinated/synchronized with breathing movement of a recipient of ventilation. Measurement/imaging/treatment performed using associated device of the system may thus be coordinated or synchronized with relatively predictable breathing movements.

In an example embodiment, the breathing pattern communication unit is operable to in use transmit the trigger signal to an associated device according to the breathing pattern such that operation of the associated device is performed in response to the trigger signal at a predetermined instance in the breathing pattern. In an example embodiment, the breathing pattern communication unit is operable to receive status information from an associated device and to adapt the breathing pattern in response thereto. In an example embodiment, the breathing pattern communication unit is operable in use to receive status information from an associated device and to adapt transmission of the trigger signal in response thereto, for example to inhibit or delay transmission of the trigger signal.

In an example embodiment, the breathing pattern controller is configured to cause the ventilator to regulate a depth and/or volume of breathing according to the breathing pattern. In an example embodiment, the breathing pattern controller is configured to cause the ventilator to regulate a rate of breathing according to the breathing pattern. In an example embodiment, the breathing pattern comprises a greater regularity in frequency and/or greater inflation volume than spontaneous breathing. In another example embodiment, the breathing pattern comprises a reduced frequency and/or lower inflation volume than spontaneous breathing. In yet further example embodiments, the breathing pattern comprises a pattern of other combinations of frequency and inflation volume, each being independently controlled to be either above or below those expected according to spontaneous breathing for a recipient of ventilation. In an example embodiment, the breathing pattern controller is configured to cause the ventilator to regulate breath holding according to a breathing pattern.

In an example embodiment, the breathing pattern controller is configured to cause the ventilator to actively regulate inhalation according to the breathing pattern. In an example embodiment, the breathing pattern controller is configured to cause the ventilator to regulate exhalation according to the breathing pattern. In an example embodiment, the breathing pattern controller is configured to cause the ventilator to inflate the chest of a recipient of the ventilation during a breath hold. In an example embodiment, the breathing pattern controller is configured to cause the ventilator to inflate the chest of a recipient of the ventilation during a breath hold that is prolonged relative to a pause in spontaneous breathing. In an example embodiment, the breathing pattern controller is configured to cause the ventilator to inflate the chest of a recipient of the ventilation during a breath hold that is prolonged, for example for more than 30 seconds, or more than 1, 3, or 5 minutes.

In an example embodiment, the breathing pattern comprises duration and/or depth and/or volume and/or rate of inhalation, wherein duration and/or depth and/or volume and/or rate of inhalation may be pre-defined and/or set by a user and/or set by a device and/or set dynamically. In an example embodiment, the breathing pattern comprises a plurality of inhalations, for example, repeated or successive inhalations or breathing in.

In an example embodiment, the breathing pattern comprises breath duration and/or depth and/or volume and/or rate of exhalation, wherein breath duration and/or depth and/or volume and/or rate of exhalation may be pre-defined and/or set by a user and/or set dynamically.

In an example embodiment, the breathing pattern comprises a plurality of inhalations exhalations, for example, repeated or successive exhalations or breathing out.

In an example embodiment, the breathing pattern comprises a pause, for example, a breath hold. In an example embodiment, the breathing pattern comprises pause, wherein duration of the pause may be pre-defined and/or set by a user and/or set by a device and/or set dynamically. In an example embodiment, the breathing pattern comprises a plurality of pauses, for example, repeated or successive breath holds.

In an example embodiment, the breathing pattern comprises regular breathing, for example, a normal breathing pattern. In an example embodiment, the breathing pattern comprises an inhalation, an exhalation and a pause. In an example embodiment, the breathing pattern comprises a plurality of inhalations, exhalations and pauses.

In an example embodiment, the breathing pattern comprises a breath hold with chest deflation, for example, a single prolonged breath with chest deflating. In an example embodiment, the breathing pattern comprises a plurality of breath holds with chest deflation, for example, repeated or successive single prolonged breaths with chest deflating. In an example embodiment, the breathing pattern comprises an inhalation, a breath hold with chest deflation and an exhalation or inhalation. In an example embodiment, the breathing pattern comprises a plurality of inhalations, breath holds with chest deflation and exhalations or inhalations.

In an example embodiment, the breathing pattern comprises a breath hold without chest deflation, for example, a single prolonged breath without chest deflating. In an example embodiment, the breathing pattern comprises a plurality of breath holds without chest deflation, for example, repeated or successive single prolonged breaths without chest deflating. In an example embodiment, the breathing pattern comprises an inhalation, a breath hold without chest deflation and an exhalation. In an example embodiment, the breathing pattern comprises a plurality of inhalations, breath holds without chest deflation and exhalations.

In an example embodiment, the breathing pattern controller is configured to operate the ventilator according to breathing feedback received at the breathing pattern controller. In an example embodiment, the breathing pattern controller is configured to dynamically set one or more aspects of the breathing pattern. In an example embodiment, the breathing pattern controller is configured to dynamically adjust one or more parameters of the breathing pattern dynamically on a continuous feedback basis.

In an example embodiment the ventilator comprises a positive pressure ventilator.

According to another aspect of the invention, there is provided a ventilator for use in the ventilator system as described above.

According to another aspect of the invention there is provided a breathing pattern controller configured for use in the ventilator system as described above.

According to another aspect of the invention there is provided a breathing pattern communication unit configured for use in the ventilator system as described above.

According to another aspect of the invention there is provided an associated device configured for use in the ventilator system as described above.

According to another aspect of the invention there is provided a method of operation of an associated device, comprising receiving a trigger signal from a breathing pattern communication unit of a ventilator system, the trigger signal being transmitted according to breathing pattern of the ventilator system; and operating the associated device in response thereto. Suitably, the method is performed by an associated device and/or ventilator system as described above.

As set out above, apparatus and methods according to example embodiments may be provided for a recipient of ventilation who is conscious and unsedated, to provide a more regular breathing pattern during operation of an associated device for imaging and/or treatment than may be achieved with spontaneous breathing.

As set out above, apparatus and methods according to example embodiments enable driving an imaging or treatment machine so as to anticipate a position of a part of the body of interest, for example a position of a tumour or healthy tissue.

As set out above apparatus and methods according to example embodiments are operable to enable a short, or a prolonged breath-hold in which a ventilator inflates the chest of a patient during breath-holding in order to prevent the chest from deflating during breath-holding.

According to the present invention there is provided an apparatus as set forth in the appended claims. Other features of the invention will be apparent from the dependent claims, and the description which follows.

### Brief Introduction to the Drawings

For a better understanding of the invention, and to show how embodiments of the same may be carried into effect, reference will now be made, by way of example, to the accompanying diagrammatic drawings in which:
Figure 1 shows a schematic diagram of a ventilator system according to an example embodiment;
Figures 2A - 2C show schematic breathing patterns according to example embodiments;
Figure 3 shows a schematic diagram of a ventilator system according to an example embodiment;
Figure 4 shows a schematic diagram of a ventilator system according to another example embodiment;
Figure 5 shows a schematic diagram of a ventilator system according to yet another example embodiment; and
Figure 6 shows a schematic diagram of a ventilator system according to still yet another example embodiment.

### Description of Example Embodiments

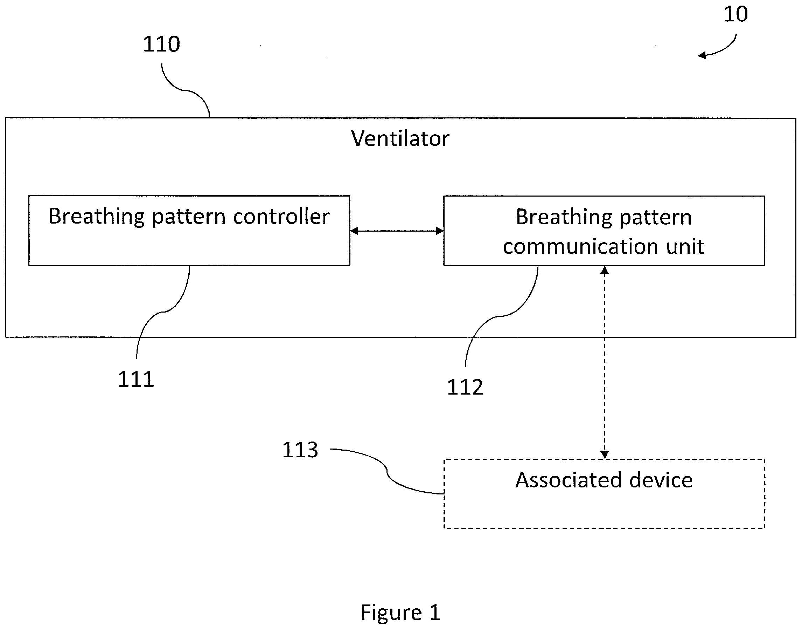

Figure 1 shows ventilator system 10 according to an example embodiment, comprising a ventilator 110, a breathing pattern controller 111 and a breathing pattern communication unit 112. The breathing pattern controller 111 is arranged to operate the ventilator 110 according to a breathing pattern stored therein. In this example embodiment the ventilator is a positive pressure ventilator arranged to provide forced mechanical ventilation. In example embodiments the breathing pattern may for example be stored in computer memory, on a hard disk, on a computer-readable medium, or communicated to the breathing pattern controller 111 on a computer network. As will be appreciated the breathing pattern may be represented numerically, diagrammatically or in writing. Figure 1 also shows an associated device 113 as part of the system. Operation of the ventilator system of Figure 1 will be described in more detail below, following an introduction to example breathing patterns which may be used by the breathing pattern controller 111. The breathing pattern communication unit 112 communicates with the associated device 113, for example providing in this embodiment two-way communication of the breathing pattern and associated trigger or monitoring signals using any suitable communication medium and protocol.

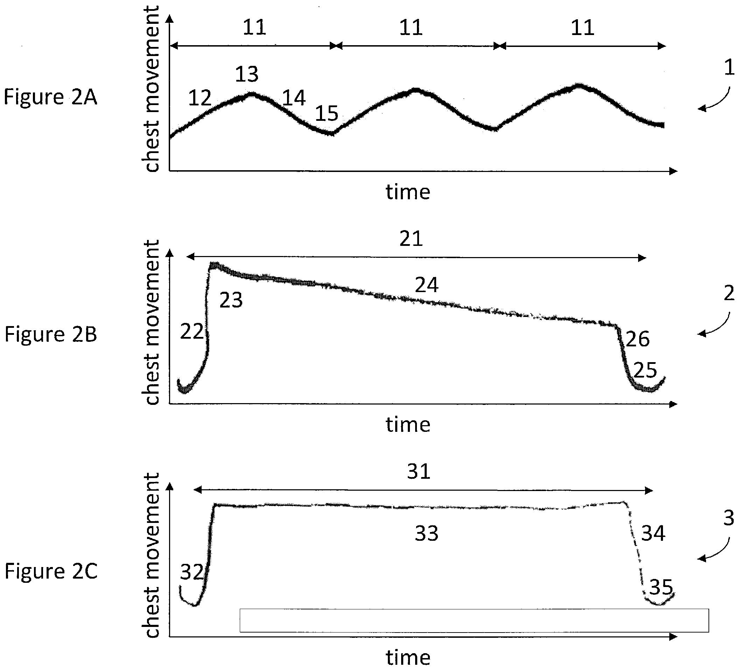

Figure 2A shows a breathing pattern 1, comprising three successive regular breathing cycles 11 of regular breathing, according to an example embodiment, in which a chest movement is plotted against time. A regular breathing cycle 11 comprises an inhalation 12 during which a chest position changes positively at an approximately constant rate, a pause 13 during which there is little or no chest movement, an exhalation 14 during which a chest position changes negatively at an approximately constant rate and a pause 15 during which there is little or no chest movement. The regular breathing cycle 11 may be repeated one or more times.

Figure 2B shows a breathing pattern 2, comprising a single prolonged breath hold with chest deflating cycle 21, according to an example embodiment, in which a chest movement is plotted against time. A single prolonged breath hold with chest deflating cycle 21 comprises a relatively faster inhalation 22 during which a chest position changes positively at an approximately constant and relatively faster rate, a pause 23 during which there is little or no chest movement, a relatively slower deflation 24 during which a chest position naturally changes negatively at an approximately constant and relatively slower rate, a relatively faster exhalation 26 during which a chest position changes negatively at an approximately constant and relatively faster rate and a pause 25 during which there is little or no chest movement. Single prolonged breath hold with chest deflating cycle 21 may be repeated one or more times. According to another example embodiment (not shown), relatively faster exhalation 26 of breathing pattern 2 is replaced with a relatively faster inhalation during which a chest position changes positively at an approximately constant and relatively faster rate, related to relatively faster inhalation 22.

Figure 2C shows breathing pattern 3, comprising a single prolonged breath hold without chest deflating cycle 31, according to an example embodiment, in which a chest movement is plotted against time. A single prolonged breath hold without chest deflating cycle 31 comprises in this example a number of distinct portions. Firstly there is provided a relatively faster inhalation 32 during which a chest position changes positively at an approximately constant and relatively faster rate. Next, there is a relatively longer pause 33 during which there is little or no chest movement because the ventilator 110 provides inflation to oppose natural deflation. After the breath hold with opposed deflation, a relatively faster exhalation 34 during which a chest position changes negatively at an approximately constant and relatively faster rate and a pause 35 during which there is little or no chest movement are provided. Single prolonged breath hold with chest deflating cycle 31 may be repeated one or more times. According to another example embodiment (not shown), relatively faster exhalation 34 of breathing pattern 3 is replaced with a relatively faster inhalation during which a chest position changes positively at an approximately constant and relatively faster rate, related to relatively faster inhalation 32.

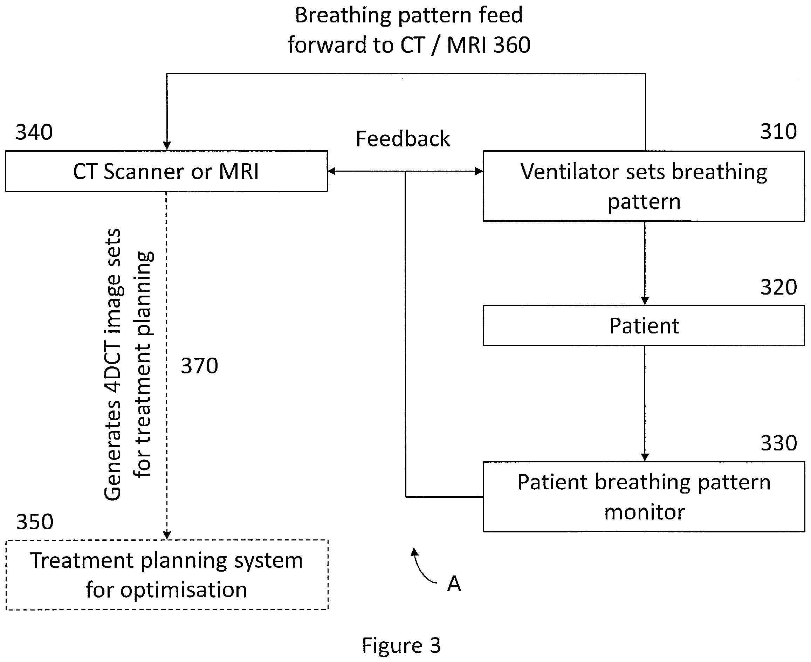

Figure 3 shows a schematic diagram of a ventilator system A comprising ventilator 310 communicatively coupled to imaging apparatus 340, for example a CT scanner or an MRI device. The ventilator 310 sets a breathing pattern such as the breathing pattern 1,2,3 of Figure 2 for a recipient of the ventilation, specifically a patient 320. The ventilator 310 passes a breathing pattern 360 to the imaging apparatus 340, including a trigger signal that corresponds to a predetermined point in the breathing cycle at which the patient 320 is still, without breathing-related movement. A patient breathing pattern monitor 330 is communicatively coupled to the patient 320 and is also communicatively coupled to the imaging apparatus 340. The imaging apparatus 340 acquires information from the patient 320 (for example data for a CT scan, data for a MRI) according to the breathing pattern fed from ventilator 310, using the trigger signal to operate its imaging functionality. Optionally, the ventilator system A is configured so that the imaging apparatus 340 further generates, for example, 4DCT image sets for treatment planning 370, which may be used by treatment planning system for optimisation 350. Information from the patient breathing pattern monitor 330 is communicated to imaging apparatus 340, for example, for feedback control of imaging apparatus 340 according to the information from patient breathing pattern monitor 330.

Although in this embodiment the patient breathing pattern monitor 330 is shown as a separate unit to the ventilator 310, it will be appreciated that the ventilator 310 may provide this functionality itself according to various known techniques. According to another embodiment, the ventilator 310 is bi-directionally communicatively coupled to the imaging apparatus 340. In such embodiments the imaging apparatus 340 is arranged to communicate information to ventilator 310, for example, for feedback control of ventilator 310 according to the information from imaging apparatus 340, either directly or by feeding through information from an external patient breathing pattern monitor 330.

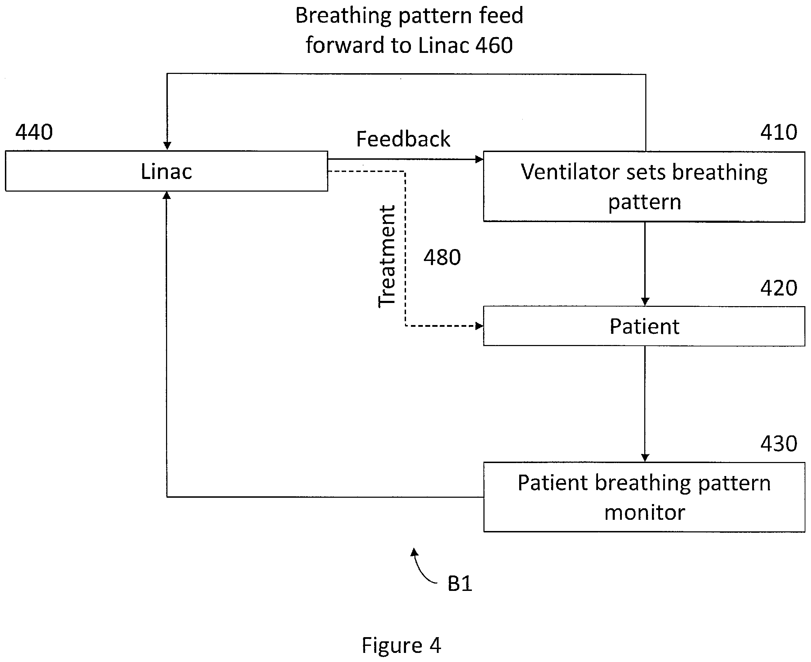

Figure 4 shows a schematic diagram of a ventilator system B1 comprising ventilator 410 communicatively coupled to treatment apparatus 440 comprising a LINAC. The ventilator 410 sets a breathing pattern such as the breathing pattern 1,2,3 of Figure 2 for a recipient of the ventilation, specifically a patient 420. The ventilator 410 passes information on the breathing pattern 460, including a trigger signal, to the treatment apparatus 440. The patient breathing pattern monitor 430 is communicatively coupled to patient 420 and is also communicatively coupled to the treatment apparatus 440. The treatment apparatus operates to provide treatment of patient 420 according to the trigger signal from the ventilator 410. Information from the patient breathing pattern monitor 430 is communicated to the imaging apparatus 440, for example, for feedback control of imaging apparatus 440 according to the information from patient breathing pattern monitor 430. According to another embodiment, the ventilator 410 is bi-directionally communicatively coupled to treatment apparatus 440. In such embodiments the treatment apparatus 440 is arranged to communicate information to the ventilator 410, for example, for feedback control of ventilator 410 according to the information from the imaging apparatus 440 by feeding through information from the patient breathing pattern monitor 430. Treatment of the patient 420 can then take place at a predetermined point in the breathing pattern. For example, the trigger signal is timed to operate the treatment function of the treatment apparatus 440 to coincide with a portion of the breathing pattern at which the patient's lungs are held still under the effect of ventilation from the ventilator 410, in order to improve the accuracy of the treatment.

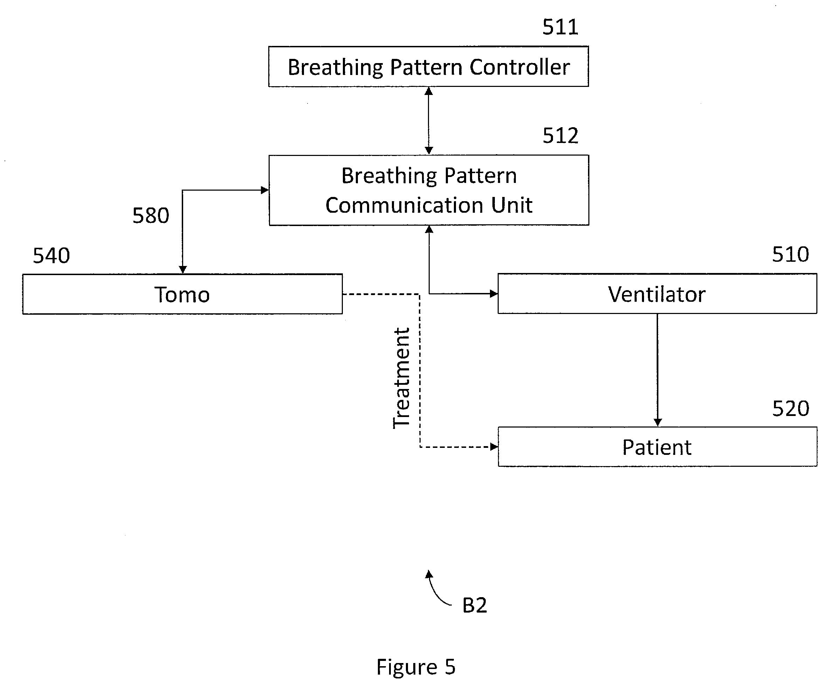

Figure 5 shows a schematic diagram of a ventilator system B2 comprising a ventilator 510 and a treatment apparatus 540 comprising a tomographic radiotherapy device (referred to as a 'tomo'). The ventilator 510 in this embodiment is configured to operate with an external breathing pattern controller 511 and a breathing pattern communication unit 512. The breathing pattern controller 511 operates the ventilator 510 according to the breathing pattern set for a recipient of the ventilation, specifically a patient 520, such as the breathing pattern 1,2,3 of Figure 2 for example. The breathing pattern communication unit 512 is configured to transmit to the tomo 540 a trigger signal 580 for treatment of patient 520, the trigger signal being timed to operate the treatment apparatus 540 to coincide with a portion of the breathing pattern at which the patient's lungs are held still under the effect of ventilation from the ventilator 510, in order to improve the accuracy of the radiotherapy. The treatment apparatus 440 is also arranged to provide feedback to the breathing pattern communication unit 512 which can be used and passed on to the ventilator 510 as appropriate.

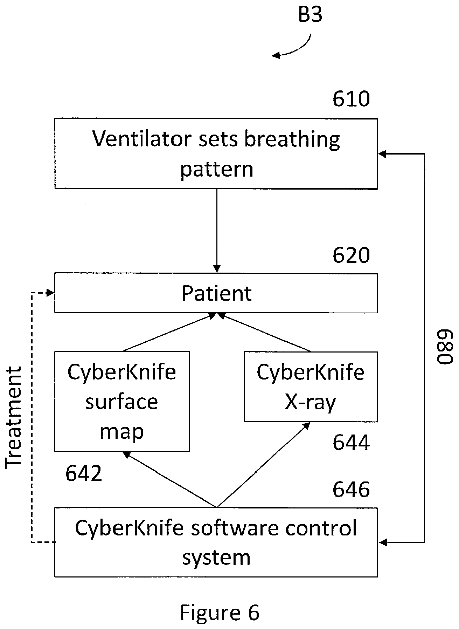

Figure 6 shows a schematic diagram of a ventilator system B3 comprising a ventilator 610 and a treatment apparatus comprising a robotic radiosurgery tool such as a CyberKnife™ surgical apparatus (CyberKnife is a trade mark of Accuray Incorporated in the United States and other countries). The ventilator 610 sets a breathing pattern for a recipient of the ventilation, specifically a patient 620, such as the breathing pattern 1,2,3 of Figure 2 using a breathing pattern controller as described in the example embodiment of Figure 1. The control system 646 for the treatment apparatus comprising a CyberKnife surface map 642 or CyberKnife X-ray 644 receives a trigger signal 680 transmitted by a breathing pattern communication unit as described in the example embodiment of Figure 1. Treatment of the patient 620 according to the breathing pattern that is used to operate the ventilator 610 can then take place using the treatment apparatus at a predetermined point in the breathing pattern by operating the treatment apparatus at the right time. The treatment apparatus is also arranged to provide feedback to the ventilator 510 and its breathing pattern controller.

As set out above, a ventilator system according to the example embodiments provides a means of ventilating a recipient of the ventilation, for example a patient, according to a predictable breathing pattern, and enables a measurement and/or treatment of the patient to be made at the most appropriate point in the ventilated breathing pattern. It becomes possible to impose a regular or otherwise clinically efficacious breathing pattern in conscious, unsedated patients and to trigger an associated device using the trigger signal, to capture images or deliver radiotherapy treatment during the breathing pattern. In example embodiments the transmission of the trigger signal is timed to coincide with the ventilator imposing a counter inflation during a prolonged breath-hold, so the chest of the patient does not deflate and the associated device is triggered to capture images or deliver radiotherapy treatment during the breath-hold.

Further, since the patient is not required to have conscious control over breathing, such a ventilator system may be suitable for those patients with reduced lung function, or with other complicating factors which prevent effective breath holding. The systems described may be applicable for various imaging techniques, and for treatment of e.g. cancer patients. This reduces the need for pharmacological interventions, while maintaining a more regular breathing pattern throughout the imaging and/or treatment so that better outcomes may be achieved than if the techniques were to be performed with spontaneous or natural breathing of the patient.

These advantages enable associated devices such as treatment machines to be used with a ventilator as part of the system that is configured to drive the treatment machine to anticipate a position of a tumour and/or healthy tissue and to apply treatment in only the desired areas. In particularly preferred examples, the ventilator system according may be used to inflate a lung or both lungs of a cancer patient during prolonged single breath holds (for example, a single breath hold comprising a duration of for example 30 seconds to as much as 1, 3 or 5 minutes) to prevent or reduce a deflation of a chest of the patient that may occur naturally and/or spontaneously during the breath hold. It is envisaged that using current technology the system will operate with a positive pressure ventilator, but it is to be understood that in certain circumstances negative pressure ventilators may also be employed.

Although a few preferred embodiments have been shown and described, it will be appreciated by those skilled in the art that various changes and modifications might be made without departing from the scope of the invention, as defined in any appended claims.

## Claims

1. A ventilator system (10) comprising:
a ventilator (110) operable to provide forced mechanical ventilation;
a breathing pattern controller (111) configured to operate the ventilator (110) according to a breathing pattern stored therein; and
a breathing pattern communication unit (112) operable to in use transmit a trigger signal, according to the breathing pattern;
**characterised by**:
wherein the breathing pattern controller (111) is configured to cause the ventilator (110) to actively regulate chest deflation according to the breathing pattern; and wherein the breathing pattern communication unit (112) is operable to in use transmit the trigger signal, according to the breathing pattern, such that the trigger signal is synchronised with the breathing pattern at a point where the breathing pattern controller (111) operates the ventilator (110) to impose a counter inflation to prevent chest deflation in a recipient of the ventilation.

2. The ventilator system (10) of claim 1, wherein the breathing pattern communication unit (112) is operable to in use transmit the trigger signal, according to the breathing pattern, such that the trigger signal is transmitted at a predetermined point in the breathing pattern.

3. The ventilator system (10) of claim 2, wherein the breathing pattern communication unit (112) is operable to in use transmit the trigger signal, according to the breathing pattern, such that the trigger signal is transmitted at the predetermined point in the breathing pattern on a cyclical basis.

4. The ventilator system (10) of any preceding claim, wherein the breathing pattern communication unit (112) is operable to in use transmit the trigger signal, according to the breathing pattern, such that the trigger signal is synchronised with the breathing pattern at a point where the breathing pattern controller (111) operates the ventilator (110) to hold the recipient of ventilation still.

5. The ventilator system (10) of any preceding claim, wherein the breathing pattern communication unit (112) is operable to in use transmit the trigger signal, according to the breathing pattern, such that the trigger signal is synchronised with the breathing pattern at a point where the breathing pattern controller (111) operates the ventilator (110) to impose a counter inflation to prevent chest deflation in the recipient of the ventilation during a period of breath-holding.

6. The ventilator system (10) of any preceding claim, further comprising an associated device (113), wherein the breathing pattern communication unit (112) is configured to transmit the trigger signal and/or the breathing pattern to the associated device (113).

7. The ventilator system (10) of claim 6, wherein the breathing pattern communication unit (112) is configured to transmit the breathing pattern to the associated device (113) that comprises a medical device in the form of: an imaging device; a radiotherapy device; or a surgical device.

8. The ventilator system (10) of claim 6 or 7, wherein the associated device (113) is arranged in use to receive the trigger signal and/or the breathing pattern and to operate in response thereto.

9. The ventilator system (10) of any one of claims 6 through 8, wherein the associated device (113) is arranged in use to receive the trigger signal and/or the breathing pattern and to operate in response thereto, in accordance with movement of the recipient of the ventilation that is produced by operation of the ventilator (110).

10. The ventilator system (10) of any one of claims 6 through 9, wherein the breathing pattern communication unit (112) is operable to in use transmit the trigger signal to the associated device (113) according to the breathing pattern such that operation of the associated device (113) is performed in response to the trigger signal at a predetermined instance in the breathing pattern.

11. The ventilator system (10) of any one of claims 6 through 10, wherein the breathing pattern communication unit (112) is arranged to receive feedback from the associated device (113), the feedback comprising one or more of: patient treatment/imaging feedback; breathing monitoring feedback, and to communicate the feedback to the breathing pattern controller (111), or to the ventilator (110) in the ventilator system (10).

12. The ventilator system (10) of any one of claims 6 through 11, wherein the breathing pattern communication unit (112) is operable to receive status information from the associated device (113) and to adapt the breathing pattern and/or transmission of the trigger signal in response thereto.

13. The ventilator system (10) of any preceding claim, wherein the breathing pattern controller (111) is configured to operate the ventilator (110) according to breathing feedback received at the breathing pattern controller (111) in order to dynamically adjust one or more parameters of the breathing pattern on a continuous feedback basis.

14. The ventilator system (10) of any preceding claim, wherein the breathing pattern controller (111) is configured to cause the ventilator (110) to inflate the chest of the recipient of the ventilation during a breath hold that is prolonged relative to a pause in spontaneous breathing.

15. A method of operating of an associated device (113), comprising:
receiving a trigger signal from a breathing pattern communication unit (112) of a ventilator system (10) according to any of claims 1 to 14, the trigger signal being transmitted according to breathing pattern of the ventilator system (10); and
operating the associated device (113) in response thereto.

## Patentansprüche

1. Ein Beatmungssystem (10), das Folgendes umfasst:
ein Beatmungsgerät (110) für mechanische Fremdbeatmung;
eine Atemmuster-Steuereinheit (111), die das Beatmungsgerät (110) gemäß dem gespeicherten Atemmuster betreibt; und
eine Atemmuster-Kommunikationseinheit (112) zum Übermitteln eines Auslösesignals, gemäß dem Atemmuster;
die sich durch Folgendes auszeichnet:
wobei die Atemmuster-Steuereinheit (111) so konfiguriert ist, dass sie das Beatmungsgerät (110) dazu bringt, gemäß dem Atemmuster das Luftablassen aus der Brust aktiv zu regulieren; und wobei die Atemmuster-Kommunikationseinheit (112) gemäß dem Atemmuster das Auslösesignal übermittelt, sodass das Auslösesignal mit dem Atemmuster an einem Punkt synchronisiert wird, und zwar wenn die Atemmuster-Steuereinheit (111) das Beatmungsgerät (110) betreibt, um eine entgegengesetzte Luftaufnahme zu erzwingen, um bei einem Empfänger der Beatmung das Luftablassen aus der Brust zu verhindern.

2. Das Beatmungssystem (10) aus Anspruch 1, wobei die Atemmuster-Kommunikationseinheit (112) gemäß dem Atemmuster das Auslösesignal übermittelt, sodass das Auslösesignal an einem bestimmten Punkt des Atemmusters übermittelt wird.

3. Das Beatmungssystem (10) aus Anspruch 2, wobei die Atemmuster-Kommunikationseinheit (112) das Auslösesignal übermittelt, gemäß dem Atemmuster, sodass das Auslösesignal an einem bestimmten Punkt des Atemmusters auf zyklischer Basis übermittelt wird.

4. Das Beatmungssystem (10) aus den vorherigen Ansprüchen, wobei die Atemmuster-Kommunikationseinheit (112) gemäß dem Atemmuster das Auslösesignal übermittelt, sodass das Auslösesignal mit dem Atemmuster an einem Punkt synchronisiert wird, an dem die Atemmuster-Steuereinheit (111) das Beatmungsgerät (110) betreibt, um den Empfänger der Beatmung stillzuhalten.

5. Das Beatmungssystem (10) der vorherigen Ansprüche, wobei die Atemmuster-Kommunikationseinheit (112) gemäß dem Atemmuster das Auslösesignal übermittelt, sodass das Auslösesignal mit dem Atemmuster an dem Punkt synchronisiert wird, an dem die Atemmuster-Steuereinheit (111) das Beatmungsgerät (110) betreibt, um während dem Atemanhalten bei dem Empfänger der Beatmung ein Luftablassen aus der Brust zu vermeiden.

6. Das Beatmungssystem (10) der vorherigen Ansprüche, das des Weiteren ein verbundenes Gerät (113) umfasst, wobei die Atemmuster-Kommunikationseinheit (112) dazu konfiguriert ist, das Auslösesignal und/oder das Atemmuster an das verbundene Gerät (113) zu übermitteln.

7. Das Beatmungssystem (10) aus Anspruch 6, wobei die Atemmuster-Kommunikationseinheit (112) dazu konfiguriert ist, das Atemmuster an das verbundene Gerät (113), welches ein medizinisches Gerät in Form eines Bilderfassungsgeräts, eines Strahlentherapie-Geräts oder eines chirurgischen Geräts umfasst, zu übermitteln.

8. Das Beatmungssystem (10) aus Anspruch 6 oder 7, wobei das verbundene Gerät (113) das Auslösesignal und/oder Atemmuster empfängt und entsprechend funktioniert.

9. Das Beatmungssystem (10) aus den Ansprüchen 6 - 8, wobei das verbundene Gerät (113) das Auslösesignal und/oder Atemmuster empfängt und entsprechend funktioniert, gemäß der Bewegung des Empfängers der Beatmung, die durch den Betrieb des Beatmungsgeräts (110) herbeigeführt wird.

10. Das Beatmungssystem (10) aus den Ansprüchen 6 - 9, wobei die Atemmuster-Kommunikationseinheit (112) das Auslösesignal an das verbundene Gerät (113) gemäß dem Atemmuster übermittelt, sodass das verbundene Gerät (113) an einem bestimmten Punkt des Atemmusters als Antwort auf das Auslösesignal funktioniert.

11. Das Beatmungssystem (10) der Ansprüche 6 - 10, wobei die Atemmuster-Kommunikationseinheit (112) Feedback von dem verbundenen Gerät (113) erhält, wobei das Feedback eine oder mehrere der folgenden Informationen umfasst: Feedback über die Behandlung des Patienten/die Bilderfassung; Feedback über die Atemüberwachung, und das Feedback der Atemmuster-Steuereinheit (111) oder dem Beatmungsgerät (110) im Beatmungssystem (10) mitteilt.

12. Das Beatmungssystem (10) aus den Ansprüchen 6 - 11, wobei die Atemmuster-Kommunikationseinheit (112) Statusinformation von dem verbundenen Gerät (113) erhält und das Atemmuster und/oder die Übertragung des Auslösesignals entsprechend anpasst.

13. Das Beatmungssystem (10) aus den vorherigen Ansprüchen, wobei die Atemmuster-Steuereinheit (111) das Beatmungsgerät (110) gemäß dem Feedback, welches die Atemmuster-Steuereinheit (111) erhalten hat, betreibt, um eine oder mehrere Parameter des Atemmusters, basierend auf kontinuierlichem Feedback, dynamisch anpasst.

14. Das Beatmungssystem (10) der vorherigen Ansprüche, wobei die Atemmuster-Steuereinheit (111) so konfiguriert ist, dass sie das Beatmungsgerät (110) dazu bringt, die Brust des Empfängers während der Beatmung bei einem Atemanhalten aufzublasen, welches entsprechend einer Pause bei einem regelmäßigen Atem verlängert wird.

15. Eine Methode zum Betrieb eines verbundenen Geräts (113), die Folgendes umfasst:
den Empfang eines Auslösesignals von einer Atemmuster-Kommunikationseinheit (112) eines Beatmungssystems (10) gemäß der Ansprüche 1 - 14, wobei das Auslösesignal gemäß dem Atemmuster des Beatmungssystems (10) übermittelt wird; und
der Betrieb des verbundenen Geräts (113) als Antwort hierauf.

## Revendications

1. Un système de ventilation (10) incluant :
un ventilateur (110) opérable pour fournir une ventilation mécanique forcée ;
un contrôleur de profils de respiration (111) configuré pour opérer le ventilateur (110) conformément à un profil de respiration enregistré dans ce contrôleur ; et une unité (112) de communication des profils de respiration pour transmettre un signal de déclenchement, conformément au profil de respiration, avec les caractéristiques suivantes :
où un contrôleur des profils de respiration (111) configuré pour que le ventilateur(110) régule dynamiquement la déflation du thorax conformément au profil de respiration ; et où l'unité intégrée de communication des profils de respiration (112) transmet un signal de déclenchement,
conformément au profil de respiration. Le signal de déclenchement est donc synchronisé au profil de respiration à un point spécifique pour que le contrôleur (111) des profils de respiration actionne le ventilateur (110) pour forcer un courant d'inflation qui prévient la déflation du thorax sous ventilation.

2. Le système de ventilation (10) de la revendication 1, où l'unité intégrée de communication des profils de respiration (112) transmet un signal de déclenchement, conformément au profil de respiration.
Le signal de déclenchement est donc transmis à un point prédéterminé du profil de respiration.

3. Le système de ventilation (10) de la revendication 2, où l'unité intégrée de communication des profils de respiration (112) transmet un signal de déclenchement, conformément au profil de respiration.
Le signal de déclenchement est donc transmis à un point déterminé du profil de respiration sur la base d'un cycle spécifique.

4. Le système de ventilation (10) de toute revendication précédente, où l'unité intégrée de communication des profils de respiration (112) transmet un signal de déclenchement, conformément au profil de respiration. Le signal de déclenchement est donc synchronisé au profil de respiration sur un point spécifique où le contrôleur des profils de respiration (111) actionne le ventilateur (110) de façon à immobiliser l'air dans le thorax sous ventilation.

5. Le système de ventilation (10) de toute revendication précédente, où l'unité de communication des profils de respiration (112) transmet un signal de déclenchement, conformément au profil de respiration. Le signal de déclenchement est donc synchronisé avec le profil de respiration sur un point spécifique où le contrôleur des profils de respiration (111) actionne le ventilateur (110) pour forcer un courant d'inflation pour prévenir la déflation du thorax sous ventilation pendant une période de respiration retenue.

6. Le système de ventilation (10) de toute revendication précédente, inclut en outre un dispositif associé (113) où l'unité de communication des profils de respiration (112) est configurée pour transmettre un signal de déclenchement et/ou le profil de respiration au dispositif associé (113).

7. Le système de ventilation (10) de la revendication 6, où l'unité de communication des profils de respiration (112) est configurée pour transmettre le profil de respiration au dispositif associé (113) qui inclut un appareil médical tel qu'un système d'imagerie, un dispositif radiothérapeutique, ou un dispositif chirurgical.

8. Le système de ventilation (10) d'une des revendications 6 ou 7, où le dispositif associé (113) est configuré pour recevoir le signal de déclenchement et/ou le profil de respiration, et pour actionner la réponse appropriée.

9. Le système de ventilation (10) de l'une quelconque des revendications 6 à 8, où le dispositif associé (113) est configuré pour recevoir le signal de déclenchement et/ou le profil de respiration, et pour actionner la réponse appropriée, conformément au mouvement du thorax sous ventilation généré par le fonctionnement du ventilateur (110).

10. Le système de ventilation (10) de l'une quelconque des revendications 6 à 9, où l'unité de communication (112) des profils de respiration transmet le signal de déclenchement au dispositif associé (113) conformément au profil de respiration, de façon à ce que le dispositif associé (113) agisse en fonction du signal de déclenchement à un point prédéterminé du profil de respiration.

11. Le système de ventilation (10) de l'une quelconque des revendications 6 à 10, où l'unité de communication des profils de respiration (112) est configurée pour recevoir le retour d'information du dispositif associé (113 (Lle retour d'information comprenant un ou plusieurs éléments parmi : le retour d'information du traitement médical/ de l'imagerie du patient ; le retour d'information de la surveillance respiratoire du patient) et pour communiquer avec le système de ventilation (10).

12. Le système de ventilation (10) de l'une quelconque des revendications 6 à 11, où l'unité de communication des profils de respiration (112) peut être configurée pour recevoir des données de statut en provenance du dispositif associé (113) et pour adapter le profil de respiration et/ou la transmission du signal de déclenchement approprié en réponse à ces données.

13. Le système de ventilation (10) de toute revendication précédente, où le contrôleur des profils de respiration (111) est configuré pour actionner le ventilateur (110), conformément au retour d'information de la respiration reçu par le contrôleur des profils de respiration (111) de manière à ajuster dynamiquement un ou plusieurs paramètres du profil de respiration selon un retour d'information continu.

14. Le système de ventilation (10) de toute revendication précédente, où le contrôleur des profils de respiration (111) est configuré pour déclencher le ventilateur (110) de manière à gonfler le thorax sous ventilation pendant une période de respiration retenue plus longue qu'une pause de respiration spontanée.

15. Une méthode de fonctionnement d'un dispositif associé (113), comprenant :
la réception d'un signal de déclenchement émis par une unité de communication des profils de respiration (112) d'un système de ventilation (10) conformément à l'une quelconque des revendications 1 à 14, le signal de déclenchement étant transmis selon le profil de respiration du système de ventilation (10) et actionnant le dispositif associé (113) en réponse à ce signal.
